Europäisches Patentamt

European Patent Office

Office européen des brevets

(19)

(11) Veröffentlichungsnummer : **0 173 941**
**B1**

(12) **EUROPÄISCHE PATENTSCHRIFT**

(45) Veröffentlichungstag der Patentschrift :
19.07.89

(21) Anmeldenummer : 85110710.2

(22) Anmeldetag : 26.08.85

(51) Int. Cl.⁴ : **C 07 C139/14**, C 07 C143/12,
C 11 D   1/28

(54) Verfahren zur Regelung des Disalzgehalts in alpha-Sulfofettsäureester-Tensiden.

(30) Priorität : 03.09.84 DE 3432324

(43) Veröffentlichungstag der Anmeldung :
12.03.86 Patentblatt 86/11

(45) Bekanntmachung des Hinweises auf die Patenterteilung : 19.07.89 Patentblatt 89/29

(84) Benannte Vertragsstaaten :
AT BE DE FR IT NL SE

(56) Entgegenhaltungen :
DE—A— 1 418 887
DE—A— 3 123 681
DE—A— 3 334 517

(73) Patentinhaber : Henkel Kommanditgesellschaft auf
Aktien
Postfach 1100 Henkelstrasse 67
D-4000 Düsseldorf-Holthausen (DE)

(72) Erfinder : Piorr, Robert, Dr.
Kieselei 22
D-4030 Ratingen-Hösel (DE)
Erfinder : Panthel, Günter, Dr.
Bollenberger Busch 7
D-5657 Haan (DE)
Erfinder : Schmid, Karl Heinz, Dr.
Stifterstrasse 10
D-4020 Mettmann (DE)
Erfinder : Colignon, Dietmar
Am Korresberg 11
D-4006 Erkrath 1 (DE)
Erfinder : Rommerskirchen, Hans Josef
Saganer Weg 21
D-4000 Düsseldorf-Eller (DE)
Erfinder : Schmidt, Wolfgang
Sandstrasse 63
D-4019 Monheim (DE)
Erfinder : Ritterbex, Horst
Beedstrasse 44
D-4000 Düsseldorf 30 (DE)

## Beschreibung

Waschaktivsubstanzen auf Basis von α-Sulfofettsäureestern bzw. ihren Salzen sind seit Jahrzehnten bekannt, und es existieren zahlreiche Vorschläge zu ihrer Herstellung (vergleiche beispielsweise US-PS 2 195 187, DE-AS 12 46 718 und DE-AS 12 48 645). Die als Waschaktivstoffe wirkenden Salze von α-Sulfofettsäureestern werden durch Sulfonierung niederer Alkylester gesättigter höherer Fettsäuren mit Schwefeltrioxid erhalten. Insbesondere werden Fettsäuremethylester, die 6 bis 28 C-Atome im Fettsäurerest aufweisen, und außer dem α-ständigen C-Atom des Feststsäurerestes keine weiteren sulfonierbaren bzw. Sulfatierbaren Gruppen aufweisen und eine Jodzahl unterhalb 5 besitzen, mit einem Schwefeltrioxid/Inertgas-Gemisch sulfoniert und das Umsetzungsprodukt neutralisiert. Da bei der Sulfonierung regelmäßig dunkelgefärbte Rohprodukte anfallen, die zur Anwendung in Wasch- und Reinigungsmitteln in dieser Form ungeeignet sind, ist die Entfärbung des rohen Sulfonierungsprodukts erforderlich. Zur Bleiche wird üblicherweise H₂O₂ und/oder Hypochlorit in wässriger Lösung eingesetzt. Bekannt ist weiterhin, daß als unerwünschtes Nebenprodukt bei dieser Sulfonierung von Fettsäureestern und der Aufarbeitung des αSulfofettsäureesterrohprodukts mit wässrigen Medien beträchtliche Mengen an Disalz der entsprechenden α-Sulfofettsäuren anfallen. Diese Disalze der α-Sulfofettsäuren sind aus mehreren Gründen unerwünscht : Sie besitzen nur beschränkte Wasserlöslichkeit und zeigen darüberhinaus schlechte Oberflächenaktivitäten. Insbesondere beeinflussen sie aber auch die Viskosität der letztlich gewünschten wässrigen Estersulfonatpasten. Ein zu hoher Gehalt der als Nebenprodukt anfallenden Disalze führt zu einer beträchtlichen Viskositätssteigerung der wässrigen Estersulfonatpasten, was zu Schwierigkeiten bei der Weiterverarbeitung des Estersulfonat-Tensides führt.

Insbesondere in jüngster Zeit ist diesem Teilaspekt der insgesamt mit zahlreichen Schwierigkeiten verbundenen Gewinnung von Tensiden auf Basis von Estersulfonaten besondere Beachtung gewidmet worden. Verwiesen wird auf DE-OS 31 23 681 und DE-OS 33 34 517. Gemäß der zuerst genannten Druckschrift wird vorgeschlagen, zur Herstellung einer hochkonzentrierten wässrigen Lösung eines Salzes von α-Sulfofettsäureestern das sulfonierte Fettsäureesterprodukt zweistufig mit einer wässrigen Alkalilösung derart zu neutralisieren, daß in einer ersten Neutralisationsstufe mit einer höherkonzentrierten wässrigen Alkalilösung (15 bis 50 Gew.-% Alkali) in Gegenwart eines Alkohols mit 1 bis 4 Kohlenstoffatomen in einer Menge von 5 bis 20 Gew.-% — bezogen auf das Gewicht des sulfonierten Produkts — bis auf einen pH-Wert 2,5 bis 4 neutralisiert wird, woraufhin in einer anschließenden Neutralisationsstufe mit einer stärker verdünnten wässrigen Alkalilösung auf einen pH-Wert von 6 bis 7 zu Ende neutralisiert wird. Vor dieser zweistufigen Neutralisation kann das Sulfonierungsrohprodukt auch gebleicht werden. Bevorzugt wird hierzu eine wässrige Lösung von H₂O₂ ebenfalls in Gegenwart eines Alkohols mit 1 bis 4 Kohlenstoffatomen eingesetzt. Das Wasserstoffperoxid soll in Form einer wässrigen Lösung mit einer Konzentration von 10 Gew.-% oder mehr verwendet werden. Der bevorzugte Alkohol ist Methanol, wenn es sich bei den Fettsäureestern um Methylester handelt. Nach diesem Vorschlag soll es möglich sein, den Disalzgehalt der entsprechenden α-Sulfofettsäuren auf 5 % oder weniger zurückzudrängen.

Die zuvor genannte DE-OS 33 34 517 beschreibt dann jedoch die Nachteile dieses Vorschlages : Die derart gewonnenen Sulfonierungsprodukte enthalten den in beträchtlichem Überschuß eingesetzten kurzkettigen Alkohol im wässrigen neutralisierten Reaktionsgut. Diese vergleichsweise hohen Mengen an freiem Alkohol sind wiederum aus einer Mehrzahl von Gründen unerwünscht. Sie stören beispielsweise bei der Aufarbeitung derartiger Tensidgemische im Rahmen der Herstellung von Waschmittelgemischen durch Sprühtrocknung, insbesondere wird dabei unerwünschtes « Pluming » ausgelöst. Die im Tensidgemisch vorliegenden freien Alkohole weisen darüberhinaus einen unerwünschten Fremdgeruch auf, so daß eine Desodorierung erforderlich ist. Zur Problemlösung schlägt die zuletzt genannte Druckschrift vor, die wässrige Bleiche und die Neutralisation der rohen α-Sulfofettsäureester in Gegenwart solcher Mengen eines niedrigen Alkohols vorzunehmen, daß eine wässrige Aufschlämmung mit 30 bis 55 Gew.-% des α-Sulfofettsäureestersalzes und — bezogen auf das Gewicht des α-Sulfofettsäureestersalzes — 5 bis 15 Gew.-% eines niedrigen Alkoholsulfats und 8 bis 40 Gew.-% des niedrigen Alkohols erhalten wird. Abschließend soll die wässrige Aufschlämmung derart eingeengt werden, daß sie 40 bis 65 Gew.-% an α-Sulfofettsäureestersalz, 2 bis 10 Gew.-% eines niedrigen Alkoholsulfats und ggf. höchstens 2 Gew.-% eines niedrigen Alkohols enthält.

Die vorliegende Erfindung geht von der überraschenden Feststellung aus, daß durch eine sehr viel zweckmäßigere Maßnahme die unerwünschte Bildung von α-Sulfofettsäure-Disalzen verhindert werden kann. Das Arbeiten mit großen Alkoholüberschüssen und das gegebenenfalls anschließende Eindampfen zur Beseitigung unerwünschter Alkoholanteile entfällt. Das erfindungsgemäße Verfahren baut auf der Erkenntnis auf, daß eine gezielt bemessene Behandlung des Rohsulfonats von Fettsäurealkylestern mit beliebigen Alkoholen in geringer, jedoch im folgenden definierter Menge zur regelbaren Senkung des Gehalts an unerwünschtem Disalz führt. Durch diese Verfahrensmaßnahme wird es darüber hinaus möglich, interessante Estergemische von α-Sulfofettsäuren herzustellen, die sich beispielsweise durch verbesserte rheologische Eigenschaften in der weiteren Verarbeitung auszeichnen können.

Gegenstand der Erfindung ist dementsprechend ein Verfahren zur Regelung und Senkung des Gehalts an α-Sulfofettsäure-Disalzen in hellfarbigen Tensiden bzw. Tensidgemischen, die durch Sulfonieren von Fettsäurealkylestern mit $SO_3$ in Molverhältnissen unter 1 : 2 und nachfolgende Aufarbeitung des Rohsulfonats in wässrigem Medium unter Salzbildung hergestellt werden. Das erfindungsgemäße Verfahren ist dadurch gekennzeichnet, daß man das Sulfonierungsprodukt vor der Behandlung mit einem wässrigen Medium mit wenigstens etwa 0,5 bis 1,3/Mol-äquivalent eines Alkohols — bezogen auf den zur α-Sulfonierung nicht verbrauchten $SO_3$-Anteil — umestert.

In einer besonders bevorzugten Ausführungsform des erfindungsgemäßen Verfahrens wird gleichzeitig die Einschränkung des Gehalts an freiem Alkohol im Reaktionsprodukt vorgesehen. Hierzu werden in der Umesterungsstufe nicht mehr als etwa 2 Mol-äquivalent der freien Alkoholkomponente eingesetzt. Insbesondere wird in dieser Stufe mit nicht mehr als etwa 1,5 Mol-äquivalent des Alkohols gearbeitet. Hierbei beziehen sich diese Mol-äquivalentangaben jeweils wieder auf den im Sulfonierungsrohprodukt vorliegenden $SO_3$-Anteil, der nicht zur α-Sulfonierung verbraucht worden ist. Diese $SO_3$-Bezugsbasis errechnet sich damit als Summe von 2 Teilbeträgen. Der eine Teilbetrag entspricht dem $SO_3$ Überschuß, der in der Sulfonierungsstufe — zur Steigerung des Umsetzungsgrades — über die zur α-Sulfonierung benötigte $SO_3$-Menge hinaus eingesetzt worden ist. Der andere Teilbetrag errechnet sich als Differenz der theoretisch benötigten $SO_3$-Menge abzüglich des in der α-Sulfonierung tatsächlich verbrauchten Anteils.

Die im jeweiligen Einzelfall zur Umesterung speziell eingesetzte Alkoholmenge kann durch die Konstitution des Alkohols mitbestimmt werden. So werden in einer bevorzugten Ausführungsform der Erfindung Alkohole, die in der Umesterungsstufe vergleichsweise hochreaktiv sind, in geringeren Mengen — innerhalb der angegebenen Bereiche — eingesetzt als weniger reaktive Alkohole. Einzelheiten hierzu werden im folgenden noch angegeben. Allgemein gilt, daß Mengen nicht über etwa 0,8 Mol-äquivalent des Alkohols — bezogen auf die zuvor erläuterte $SO_3$-Menge — zweckmäßig sein können, wobei das Arbeiten mit Alkoholmengen im Bereich von 0,9 bis 1,1 Mol-äquivalent Alkohol besonders bevorzugt sein kann.

Die Bedingungen der Umesterungsreaktion werden — insbesondere unter Anpassung an die Reaktivität des zur Umesterung eingesetzten Alkohols — so gewählt, daß das Ausmaß der zusätzlichen thermischen Belastung des Reaktionsgemisches möglicht gering gehalten wird. Auf diese Weise kann die Bildung unerwünschter zusätzlicher Verfärbungen im Reaktionsprodukt eingeschränkt oder verhindert werden. Hinreichend intensive Reaktionsbedingungen sind allerdings notwendig, um die erfindungsgemäß geforderte Umesterung zu bewirken. Allgemein gilt, daß die Umesterung bei Temperaturen von etwa 40 °C bis 150 °C, vorzugsweise bei Temperaturen nicht über 120 °C, durchgeführt wird. Besonders geeignet können Temperaturen oberhalb 60 °C vorzugsweise oberhalb 70 °C und insbesondere über 75 °C sein. Ein geeigneter Temperaturbereich liegt beispielsweise über 75 °C bis 100 °C. Die Reaktionsdauer wird in Abhängigkeit von der Reaktivität des zur Umesterung eingesetzten Alkohols, der gewählten Reaktionstemperatur und dem gewünschten Ausmaß der Senkung des Disalzgehalts bestimmt. Im allgemeinen werden wenigstens 5, insbesondere wenigstens 10 Minuten Reaktionszeit erforderlich sein, geeignet ist üblicherweise der Bereich von etwa 10 bis 30 Minuten.

Zur Auswahl der Verfahrensbedingungen im einzelnen gilt die folgende allgemeine Gesetzmäßigkeit: In der Umesterungsreaktion vergleichsweise hochreaktiv sind niedere Alkohole, insbesondere monofunktionelle niedere Alkohole mit beispielsweise 1 bis 5 C-Atomen, aber auch niedere mehrwertige Alkohole sind vergleichsweise stark reaktiv. Höhere einwertige oder mehrwertige Alkohole zeigen im allgemeinen eine geringere Reaktionsfähigkeit. Als Beispiel seien hier Fett- oder Wachsalkohole genannt. Ihre Verwendung benötigt also innerhalb des genannten Rahmens intensivere Umsetzungsbedingungen.

Als Ergebnis der Umesterungsreaktion wird der zur Umsetzung eingesetzte freie Alkohol in Esterform an die α-Sulfofettsäure gebunden. Die ursprünglich im eingesetzten Fettsäureester vorliegende Alkoholkomponente wird als Alkoholsulfat abgespalten und liegt also beispielsweise beim Einsatz von Fettsäuremethylestern als Ausgangsmaterial zur Sulfonierungsreaktion nach der Umesterung im Reaktionsgemisch als Methylsulfat vor.

Die Auswahl der Reaktionskomponenten und Arbeitsbedingungen wird im allgemeinen so getroffen, daß der Disalzgehalt im mit wässrigen Medien behandelten und neutralisierten Reaktionsprodukt kleiner 10 Gew.-% — bezogen auf Waschaktivsubstanz — liegt. Bevorzugt werden Mengen des Disalzgehalts gleich oder kleiner 5 Gew.-%, wobei es aber möglich ist, auch noch zu geringeren Werten, beispielsweise Mengen kleiner 2 Gew.-% zu kommen. Bei der Verwendung hochreaktiver Alkohole in der Umesterungsstufe liegt gleichzeitig praktisch kein nachweisbarer Alkohol in der Estersulfonatpaste vor. Bei dem Arbeiten mit weniger reaktiven Alkoholen können beschränkte Mengen des im geringen Überschuß eingesetzten freien Alkohols im Produkt toleriert werden, um zu akzeptablen Reaktionszeiten bei der Senkung des Disalzgehaltes unter die angegebenen Grenzwerte zu kommen.

Als Alkoholkomponente für den Umesterungsschritt können im Prinzip beliebige Alkohole eingesetzt werden. Geeignet sind also sowohl monofunktionelle wie auch polyfunktionelle Alkohole. In einer wichtigen Ausführungsform der Erfindung setzt man zur Umesterung den gleichen Alkohol ein, wie er im Fettsäurealkylester-Ausgangsmaterial vorliegt. Insbesondere wird hier mit aliphatischen $C_{1-3}$-Monoalkoholen und den entsprechenden Fettsäurealkylestern gearbeitet, wobei besondere Bedeutung dem Methanol und den damit korrespondierenden Fettsäuremethylestern zukommt. Da diese niederen Alkohole und insbesondere Methanol sich in der Umesterung durch hohe Reaktionsfähigkeit auszeichnen, kann die eingesetzte Menge des freien Alkohols unbedenklich auf die Menge eingeschränkt werden,

3

die dem zur α-Sulfonierung nicht verbrauchten SO₃-Anteil entspricht. Gleichzeitig kann bei vergleichsweise milderen Bedingungen von Temperatur und/oder Reaktionsdauer gearbeitet werden. Das Ergebnis der erfindungsgemäßen Umesterung ist ein α-Sulfofettsäure-Alkylester — insbesondere -Methylester — in Abmischung mit einer geringen Menge Methylsulfat, der weitgehend disalzfrei ist und keine wahrnehmbaren Mengen an freiem Alkohol enthält.

In einer besonders wichtigen Ausführungsform der Erfindung werden zur Umesterung jedoch andere Alkohole eingesetzt als sie im Fettsäureester-Ausgangsmaterial vorliegen. Auch hier lassen sich zwei grundsätzliche Gruppen unterscheiden : Die Verwendung von ein- und/oder mehrwertigen Alkoholen, die bei ihrer Umsetzung mit freiem SO₃ zur Bildung kapillaraktiver Sulfate mit Tensidcharakter befähigt sind, bzw. die Umesterung mit ein- und/oder mehrwertigen Alkoholen, die bei ihrer Umsetzung mit freiem SO₃ Sulfate ohne kapillaraktivem Charakter ausbilden.

Zu der zuletzt genannten Gruppe zählen insbesondere Alkohole mit begrenzter Kohlenstoffzahl, beispielsweise monofunktionelle Alkohole mit nicht mehr als 9 C-Atomen. Aber auch niedere Polyole, beispielsweise Ethylenglykol oder Glycerin, kommen hier in Betracht. Zur Gruppe der Alkohole, die bei ihrer Umsetzung mit freiem SO₃ zur Bildung kapillaraktiver Sulfate mit Tensidcharakter befähigt sind, zählen solche Verbindungen, die im Molekül wenigstens einen hydrophoben Rest wenigstens eine aliphatisch gebundene Hydroxylgruppe enthalten. Der in diesen Hydroxylverbindungen vorhandene hydrophobe Rest kann ein Kohlenwasserstoffrest mit wenigstens 10 Kohlenstoffatomen, insbesondere aliphatischer oder cycloaliphatischer Natur sein. Der hydrophobe Rest kann beispielsweise bis zu 30 Kohlenstoffatome besitzen.

Zu der hier betroffenen Gruppe von Alkoholen gehören beispielsweise die gesättigten Fettalkohole bzw. Fettalkoholgemische natürlichen oder synthetischen Ursprungs oder Fettsäurealkylolamide. Diese Hydroxylverbindungen können ebenso wie das Ausgangsmaterial für die Sulfonierungsstufe aus natürlich vorkommenden Fetten und Ölen gewonnen worden sein. Anstelle solcher Fettalkohole oder Fettsäurealkylolamide können beliebige andere Hydroxylverbindungen Verwendung finden, die grundsätzlich bei der Umsetzung mit freiem SO₃ zur Bildung kapillaraktiver Substanzen befähigt sind. Genannt seien hier Teilether von mehrwertigen Alkoholen mit Fettalkoholen und Teilester von mehrwertigen Alkoholen mit Fettsäuren, beispielsweise Teilether und Teilester von Ethylenglykol, Propylenglykol, Glycerin, Pentaerythrit, Mannit, Hexit sowie Teilether und Teilester von Polyethylen- und/oder Polypropylenglykolen, Polyglycerinen, Polypentaerythrit u. dgl. Vor allen Dingen sind hier diejenigen Polyglykolether zu nennen, die man durch Anlagerung von Ethylen- und/oder Propylenoxid an Fettalkohole, Fettsäuren, Fettsäureamide oder an die Teilether oder Teilester von Fettalkoholen und Fettsäuren mit 2-, 3- und mehrwertigen Alkoholen erhält.

Ganz allgemein gilt, daß die zur Umesterung eingesetzten Alkohole in einer bevorzugten Ausführungsform ihrerseits im Molekül neben der Hydroxylgruppe keine reaktiven Gruppen aufweisen, die zu unerwünschten Nebenreaktionen befähigt sind.

Als Alkoholkomponenten kommen beispielsweise in Frage : Monofunktionelle aliphatische und cycloaliphatische Alkohole mit 1 bis 30, vorzugsweise 1 bis 24 Kohlenstoffatomen wie Methanol, Ethanol, n-Propanol, i-Propanol, n-Butanol, 2-Butanol, n-Pentanol, 2-Pentanol, n-Hexanol, n-Octanol, n-Decanol, n-Dodecanol, n-Tetradecanol, n-Hexadecanol, n-Octadecanol, n-Eikosanol, n-Docosanol, 2-Hexyldecanol, 2-Octyldodecanol, 2-Dodecylhexadecanol,Oxoalkohole mit 9 bis 18 Kohlenstoffatomen, Ziegler-Alkohole mit 8 bis 20 Kohlenstoffatomen, Cyclohexanol und Methylcyclohexanole ; polyfunktionelle aliphatische und cycloaliphatische Alkohole wie Ethylenglykol, 1,2-Propylenglykol, 1,3-Propylenglykol, 1,2-Butylenglykol, 1,4-Butylenglykol, Hexamethylenglykol, Polyethylenglykole, Polypropylenglykole, Glycerin, Polyglycerine, Trimethylolethan, Trimethylolpropan, Pentaerythrit, Dipentaerythrit, Mannit, Sorbit, 1,2-Cyclohexandiol und 1,3,5-Cyclohexantriol. Die genannten Alkohole können im erfindungsgemäßen Verfahren einzeln oder im Gemisch eingesetzt werden. Fettalkohole im engeren Sinn, d. h. geradkettige aliphatische Alkohole mit 8 bis 24 Kohlenstoffatomen werden — ihrer Herkunft entsprechend — im Regelfall als Gemische eingesetzt, wobei die Zusammensetzung solcher Alkoholgemische von den natürlichen Fetten und Ölen bestimmt wird, die als Ausgangsmaterial für ihre Herstellung verwendet werden.

Weitere Beispiele für mögliche Alkoholkomponenten sind Glykolhalbether wie Methylethylenglykol, Ethylethylenglykol und Anlagerungsprodukte von 1 bis 20 Mol Ethylen- und/oder Propylenoxid an aliphatische Alkohole mit 1 bis 24 Kohlenstoffatomen, insbesondere an Fettalkohole und Fettalkoholgemische ; Glykolhalbester wie Ethylenglykolmonolaurat, Ethylenglykolmonomyristat und Propylenglykolmonostearat sowie Anlagerungsprodukte von 1 bis 20 Mol Ethylen- und/oder Propylenoxid an aliphatische Carbonsäuren mit 1 bis 24 Kohlenstoffatomen, insbesondere an Fettsäuren und Fettsäuregemische ; Glycerinteilether und -Teilester wie Glycerinmonodecylether, Glycerinmonoacetat, Glycerindiacetat, Glycerinmonopalmitat, Glycerindistearat und Ethylen- und/oder Propylenoxidaddukte dieser Glycerinderivate ; Fettsäurealkanolamide wie Laurinsäuremonoethanolamid, Laurinsäurediethanolamid, Stearinsäurediethanolamid sowie Ethylen- und/oder Propylenoxidaddukte an Carbonsäureamide, insbesondere an Fettsäureamide.

Das der Umesterung zu unterwerfende Rohsulfonat soll in der bevorzugten Ausführungsform des erfindungsgemäßen Verfahrens einen in der α-Sulfonierung nicht verbrauchten SO₃-Anteil von nicht mehr als 50 Mol.-% und vorzugsweise von nicht mehr als 25 Mol.-% — jeweils bezogen auf den gebildeten

α-Sulfofettsäureester — aufweisen. Bevorzugt ist es weiterhin, daß diese Rohsulfonate einen Sulfonierungsgrad von wenigstens 90 %, insbesondere von wenigstens 95 % und zweckmäßigerweise von 98 % oder mehr — jeweils bezogen auf den eingesetzten Fettsäureester — aufweisen.

Für die der Umesterungsstufe vorgängige Sulfonierung gelten die Angaben des Standes der Technik, verwiesen sei beispielsweise auf die Angaben der DE-AS 12 46 718 und der DE-AS 12 48 645. Als Ausgangsmaterial für diese Sulfonierung werden bevorzugt niedere Alkylester, insbesondere die Methylester von Fettsäuren eingesetzt, die beispielsweise 6 bis 28 und vorzugsweise 8 bis 18 Kohlenstoffatome enthalten. Diese Fettsäurereste entstammen bevorzugt natürlichen Fetten von Pflanzen, Land- oder Wassertieren. Sie sollen abgesehen vom α-ständigen Wasserstoffatom keine weiteren sulfatierbaren oder sulfonierbaren Gruppen, insbesondere Doppelbindungen oder alkoholische Hydroxylgruppen enthalten. Ihre Jodzahlen liegen unterhalb von 5, vorzugsweise unterhalb von 2. Die Sulfonierung erfolgt mit einem $SO_3$/Inertgas-Gemisch, das üblicherweise 2 bis 40 Volum-% $SO_3$ enthalten kann. Bei Temperaturen, die 100 °C und vorzugsweise 95 °C nicht oder nicht wesentlich überschreiten. Es kann bei konstanter Verfahrenstemperatur oder mit stufenförmiger Temperaturführung gearbeitet werden wie es in den zuvor genannten Druckschriften beschrieben ist.

An die erfindungsgemäße Umesterungsstufe schließt sich die Aufarbeitung des Reaktionsprodukts mit wässrige Medien in an sich bekannter Weise an. Hierbei handelt es sich insbesondere um die Bleiche und die Neutralisation des erfindungsgemäß umgeesterten Rohsulfonats. Die Bleiche kann in an sich bekannter Weise mit wässrigem Wasserstoffperoxid und/oder Hypochloritlösung erfolgen. Die Neutralisation kann der Bleiche vorgängig sein oder erst nach der Bleiche durchgeführt werden. Die saure Bleiche mit Wasserstoffperoxid ist beispielsweise in der DE-PS 11 79 931 beschrieben, eine Kombinationsbleiche, bei der sich an eine zunächst saure Peroxidbleiche die Neutralisierung des sulfonierten und teilgebleichten Gutes anschließt, woraufhin erneut mit Wasserstoffperoxid oder besser mit Hypochlorit eine abschließende Bleichstufe erfolgt, beschreibt die DE-AS 12 34 709.

Die Verfahrensbedingungen von Bleichstufe und/oder Neutralisation sind dabei so zu wählen, daß hier die prinzipiell mögliche Esterverseifung ausgeschlossen oder soweit wie möglich unterdrückt wird. Ohne diese Vorsichtsmaßnahmen würden die Vorteile der erfindungsgemäßen Umesterungsstufe bezüglich der Reduzierung des Disalzgehaltes wenigstens teilweise wieder verloren gehen.

Durch die erfindungsgemäße Reaktion der Rohsulfonsäureprodukte mit den Alkoholen im Sinne der geforderten Umesterung läßt sich nicht nur der Disalzgehalt gezielt senken, darüberhinaus besteht die Möglichkeit, ein breites Spektrum neuer Estersulfonat-Gemische (beispielsweise Methylestersulfonat + Estersulfonat des zur Nachveresterung eingesetzten Alkohols) mit unterschiedlichen anwendungstechnischen Eigenschaften einzustellen. Die Erfindung eröffnet damit einen interessanten Weg, wässrige Aufschlämmungen bzw. Pasten von Estersulfonaten mit hohem Gehalt an α-Sulfofettsäureestersalzen und gleichwohl niedriger Viskosität herzustellen. Diese niedrige Viskosität wird dabei einerseits durch die Reduzierung des unerwünschten Disalzes in der Estersulfonatpaste erreicht, zum anderen können aber auch gerade Sulfonatestergemische zur Viskositätssenkung in den Pasten hohen Fettstoffgehalts führen (vergleiche hierzu die Angaben der eingangs zitierten DE-OS 33 34 517).

In dem folgenden Beispiel wird zunächst eine allgemeine Verfahrensvorschrift für die Durchführung der Umesterungsreaktion im Sinne der Erfindung gegeben. Die anschließende tabellarische Zusammenstellung zeigt die jeweils zur Umesterung eingesetzten Alkoholkomponenten, die dabei eingesetzten Reaktionszeiten und Reaktionstemperaturen, das Molverhältnis des zur α-Sulfonierung nicht verbrauchten $SO_3$-Anteils im Rohsulfonat zur Menge des zur Umesterung eingesetzten Alkohols sowie die abschließend gefundenen Werte für den Gehalt an Disalz in Gew.-% bezogen auf die Waschaktivsubstanz.

Beispiele

Beispiel 1-16

In einem Fallfilmreaktor wurden 283 g (1 Mol) gehärteter Talgfettsäuremethylester (Jodzahl 0,5 ; Verseifungszahl 198) bei 90 °C mit 96 g (1,2 Mol) Schwefeltrioxid (5 Vol.-% in Luft) bei 90 °C sulfoniert. Das resultierende Reaktionsgemisch wurde anschließend 30 Minuten lang bei 90 °C gealtert. Der Sulfonierungsgrad betrug danach 98 %.

Das gealterte rohe Sulfonierungsprodukt wurde bei 90 °C unter Rühren mit 7,0 g (0,22 Mol) Methanol versetzt und 20 Minuten lang bei 90 °C weitergerührt. Anschließend wurde das Reaktionsprodukt zur Bleichung mit 16 g Wasserstoffperoxid in Form einer 35 gewichtsprozentigen wässrigen Lösung versetzt und 10 Minuten lang bei 60 °C gerührt, bevor es durch Zugabe einer 25-gewichtsprozentigen Natriumhydroxidlösung bis zum pH-Wert 7 neutralisiert wurde. In der auf diese Weise erhaltenen Lösung der neutralen Salze betrug der Disalzgehalt 5,2 Gew.-%, bezogen auf die Gesamtmenge Waschaktivsubstanz.

Der Disalzgehalt wurde durch potentiometrische Titration einer auf pH 2,5 bis 3 eingestellten wässrigen Lösung des gebleichten und neutralisierten Sulfonierungsproduktes mit Natriumhydroxidlösung unter Berücksichtigung der im nicht sulfonierten Material vorhandenen Fettsäureanteile bestimmt.

In den Beispielen 2 bis 16 wurde die oben angegebene Verfahrensvorschrift dahingehend abgewandelt, daß die Umesterung mit den in der nachfolgenden Tabelle angegegebenen Alkoholen anstelle von

**EP 0 173 941 B1**

Methanol durchgeführt wurde. In Beispiel 16 wurde zu Vergleichszwecken ein Versuch unter den in Beispiel 1 angegebenen Bedingungen, jedoch ohne Umesterung zwischen Sulfonierung und Bleiche, durchgeführt.

Die in den Beispielen 1 bis 16 gefundenen Ergebnisse sind in der nachstehenden Tabelle zusammengefaßt.

Tabelle

Umesterung roher Sulfonierungsprodukte von gehärtetem
Talgfettsäuremethylester bei 90 °C

| Beispiel | Alkoholkomponente | Reaktions-zeit (min) | $SO_3$ Überschuß: Alkohol (Mol : Mol-aquiv.) | Disalzgehalt (Gew.-%) |
|---|---|---|---|---|
| 1 | Methanol | 20 | 1 : 1 | 5,2 |
| 2 | Ethanol | 20 | 1 : 1 | 6,8 |
| 3 | n-Propanol | 20 | 1 : 1 | 6,6 |
| 4 | n-Butanol | 20 | 1 : 1 | 7,6 |
| 5 | n-Octanol | 20 | 1 : 1 | 6,0 |
| 6 | 2-Ethylhexanol | 20 | 1 : 1 | 6,4 |
| 7 | Lauryl-/Myristylalko-hol (Molverh. 3 : 1) | 20 | 1 : 1 | 5,5 |
| 8 | Ethylenglykol | 10 | 1 : 1 | 8,2 |
| 9 | Ethylenglykol | 20 | 1 : 1 | 6,1 |
| 10 | Glycerin | 10 | 1 : 1 | 9,1 |
| 11 | Glycerin | 20 | 1 : 1 | 6,6 |
| 12 | Oleylalkohol + 1 PO + 6EO | 10 | 1 : 1 | 12,0 |
| 13 | Oleylalkohol + 1 PO + 6EO | 20 | 1 : 1 | 7,6 |
| 14 | 2-Ethoxyethanol | 20 | 1 : 1 | 7,1 |
| 15 | 2-Ethoxyethanol | 20 | 1 : 0,7 | 5,3 |
| 16 | Methanol | 20 | 1 : 3 | 1,6 |
| 17 | ohne (Vergleichsversuch) | -- | - | 22,7 |

**Patentansprüche**

1. Verfahren zur Regelung und Senkung des Gehalts an α-Sulfofettsäure-disalzen in hellfarbigen Tensiden bzw. Tensidgemischen, hergestellt durch Sulfonieren von Fettsäurealkylestern mit $SO_3$ in Molverhältnissen unter 1 : 2, insbesondere von 1 : 1,1 bis 1,3, und nachfolgende Aufarbeitung des Rohsulfonats in wässrigem Medium unter Salzbildung, dadurch gekennzeichnet, daß man das Sulfonierungsprodukt vor der Behandlung mit einem wässrigen Medium mit wenigstens etwa 0,5 bis 1,3 Moläquivalent eines Alkohols — bezogen auf den zur α-Sulfonierung nicht verbrauchten $SO_3$-Anteil — umestert.

2. Verfahren nach Anspruch 1, dadurch gekennzeichnet, daß man zur gleichzeitigen Einschränkung des Gehalts an freiem Alkohol im Produkt zur Umesterung nicht mehr als etwa 0,8 Mol-äquivalent des Alkohols einsetzt und diesen vorzugsweise in Mengen von etwa 0,9 bis 1,1 Mol-äquivalent verwendet.

**Claims**

1. A process for regulating and reducing the content of α-sulfofatty acid disalts in light colored surfactants and surfactant mixtures produced by sulfonation of fatty acid alkyl esters with $SO_3$ in molar ratios below 1 : 2 and, more particularly, in molar ratios of from 1 : 1.1 to 1 : 3 and subsequent working up of the crude sulfonate in aqueous medium to form salts, characterized in that, before the treatment with an aqueous medium, the sulfonation product is transesterified with at least about 0.5 mole equivalent of an alcohol, based on the $SO_3$ which is not used for α-sulfonation.

6

2. A process as claimed in Claim 1, characterized in that, for simultaneously limiting the content of free alcohol in the product, no more than about 2 mole equivalents of the alcohol, preferably about 0.8 to 1.3 mole equivalents and, more preferably, 0.9 to 1.1 mole equivalents of the alcohol are used for transesterification.

**Revendications**

1. Procédé de régulation et de réduction de la teneur en di-sels d'acides gras $\alpha$-sulfonés dans les dérivés tensio-actifs ou les mélanges de dérivés tensio-actifs de couleur claire, fabriqués par sulfonation d'alkylesters d'acides gras avec $SO_3$ dans des rapports molaires inférieurs à 1 : 2, en particulier de 1 : 1,1 à 1,3, et traitement successif du sulfonate brut dans un milieu aqueux sous formation de sel, caractérisé en ce qu'on transestérifie le produit de sulfonation, avant le traitement avec un milieu aqueux, avec au moins environ 0,5 à 1,3 équivalent molaire d'un alcool — par rapport à la partie $SO_3$ non consommée pour l'$\alpha$-sulfonation.

2. Procédé selon la revendication 1, caractérisé en ce que, pour la limitation simultanée de la teneur en alcool libre dans le produit de transestérification, on n'utilise pas plus qu'environ 0,8 équivalent molaire de l'alcool et qu'on utilise celui-ci de préférence dans des quantités de l'ordre de 0,9 à 1,1 équivalent molaire.